# EUROPEAN PATENT SPECIFICATION

(11) **EP 1 684 838 B1**
(45) Date of publication and mention of the grant of the patent: **08.12.2010**
(21) Application number: 04794760.1
(22) Date of filing: 13.10.2004
(51) Int. Cl.: A61M 25/00, A61M 39/10

(54) **CATHETER WITH A SIDEARM FOR DELIVERY OF ANTIMICROBIAL AGENTS TO PREVENT INFECTION**
KATHETER MIT SEITENARM ZUR ABGABE VON ANTIMIKROBIELLEN MITTELN ZUR VERHINDERUNG VON INFEKTIONEN
CATHETER A BRAS LATERAL CON U POUR ADMINISTRER DES AGENTS ANTIMICROBIENS ET PREVENIR L'INFECTION

(30) Priority: 22.10.2003 US 690436
(43) Date of publication of application: 02.08.2006
(73) Proprietor: Boston Scientific Limited, St. Michael, Barbados, West Indies (BB)
(72) Inventor: TAN, Sharon, Mi, Lyn, Brighton, MA 02135 (US)
(74) Representative: Joly, Jean-Jacques
(86) International application number: PCT/US2004/033491
(87) International publication number: WO 2005/042077

(56) References cited:
- EP-A- 0 791 371
- WO-A-2004/004823
- WO-A-2004/034767
- WO-A1-2005/092421
- US-A- 5 352 215

## Description

### Field Of The Invention

The present invention is directed to an improved apparatus for prevention of nosocomial infections associated with catheters.

### Background

Catheters have become widely used in modem medicine to provide one or more lumens into a patient's body through which a wide variety of procedures may be performed or fluids may be introduced or removed from the patient. Examples include catheters through which minimally-invasive surgical procedures, such as angioplasty balloon deployment or tissue resection, may be performed; catheters for introducing therapeutic substances at desired sites within the patient; catheters for the removal and/or replacement of fluids, such has blood removal and replacement during hemodialysis; and catheters associated with activation of mechanisms for medical devices, such as activation of control cables or application of pneumatic pressure to inflate balloons or expand stents at target locations within the patient.

Inherent with the use of medical devices placed within a patient is the risk of infection from the medical device. While great strides have been made in the last century in preventing infection during surgical procedures, this risk has not been entirely eliminated. For example, it has been estimated that central venous catheters account for more than 90 percent of all nosocomial catheter-related blood stream infections.

In the case of central venous catheters, characteristically, at an infected site a large number of microorganisms are adherent on the catheter, where there is an interaction between the pathogen and the catheter microsurface. Once so infected, the microorganisms adhere to the catheter and rapidly become encased in a polysaccharide matrix or biofilm which protects the microorganisms from the natural defenses of the host. While there have been recent developments of central venous catheters to attempt to reduce the incidence of nosocomial catheter-based infections, such as the use of catheters with chlorohexidine and silver sulfadiazine, or with a combination of minocycline and rifampin, such infections have yet to be eliminated.

The use of antimicrobial agent-bearing intervention devices has been proposed for the management of nosocomial blood stream infections. Antimicrobial agents such as iodine, trilosan and ageon have long been used in medical practice as disinfectants, with iodine having been discovered to be one of the most effective antiseptics in the 1870s. Recently, iodine-bearing formulations have been developed that may be applied to, or incorporated into, medical devices to provide controlled in-situ release of iodine as an antimicrobial agent. One potential application of such formulations is an iodine-bearing polymeric rod that can be inserted into a catheter, where the rod delivers iodine to the catheter in order to manage catheter-based nosocomial bloodstream infections. In this example, an iodine-bearing polymeric intervention device is placed within an indwelling catheter. As a result, elemental iodine may be released to diffuse to the catheter wall, and if the catheter wall material is semi-permeable, to diffuse through the catheter wall to the exterior surface of the catheter. Thus, the iodine may be made available to eliminate micro-organisms on both the inner and outer micro-surfaces of the catheter.

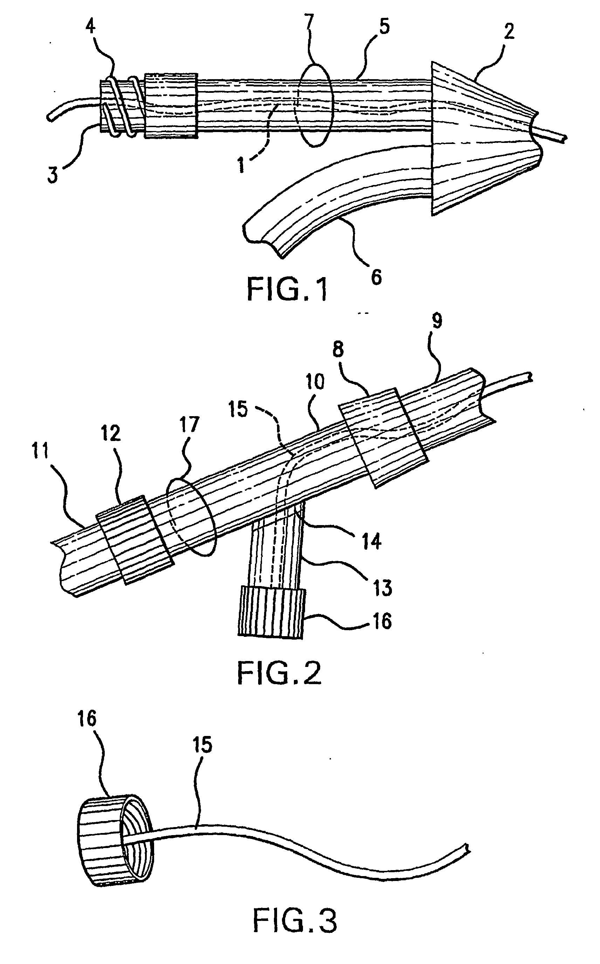

Notwithstanding the advantages in catheter-based nosocomial blood stream infection management offered by the use of an iodine-bearing polymeric device, existing catheter designs for certain applications, such as hemodialysis catheters, do not permit the use of such iodine-bearing devices without undesirable complications and difficulties. Figure 1 illustrates the use of an iodine-bearing flexible polymer rod 1, with an existing design hemodialysis catheter 2. With this catheter design, an iodine-bearing rod is held between the fingers and inserted into the catheter's proximal inlet or outlet ports (insertion into inlet port 3 shown in Fig. 1) and gently threaded through the catheter lumen. Once indwelling in the catheter, the environmental conditions in the catheter cause iodine to be released from the iodine-bearing rod to the catheter, thereby delivering iodine to the catheter from the iodine-bearing rod. Before hemodialysis may proceed, the iodine-bearing rod must be removed in order to permit connection of the catheter inlet and outlet lumens to the dialysis equipment (in Fig. 1, connection may be made via threaded inlet connection 4 at the proximal end of catheter inlet lumen extension tube 5, and a similar threaded connection at the proximal end of outlet lumen 6; outlet proximal end and dialysis equipment not shown in Fig.1). Further, following completion of hemodialysis, the catheter inlet and outlet may be disconnected from the dialysis equipment, and new iodine-bearing rods may be reinserted into the catheter lumens. Because the proximal ends of the catheter lumens must be open during the rod insertion and removal steps, air entering the lumens may cause blood clots to be formed in the catheter, which can hinder insertion of the soft, flexible iodine-bearing rod and otherwise complicate catheter use. Moreover, while the proximal ends of the catheter lumens are normally held closed by clamps 7 when the inlet or outlet ends of the catheter are open to the atmosphere, during the periods when the iodine-bearing rods are being inserted or withdrawn from the lumens the clamps must be released to permit the rods to pass, which potentially allows undesirable back-bleeding of fluids (in the present hemodialysis example, blood) out from the catheter lumens.

EP 0 791 371 discloses, in one example, a γ-connector for medical infusion/transfusion lines, catheters, and the like, in which the connector includes a protection valve for automatically stopping fluid flow when a connecting line or other device is disconnected from the connector. This document discloses the features of the preamble of claim 1.

US 5 352 215 discloses a Y-adapter having a side arm that smoothly merges into a main lumen, for facilitating the insertion and operation of devices therethrough.

### Summary Of The Invention

There is a need for a system for infection management in which an infection management device may be inserted, indwell, and be removed from a catheter in a patient's body without either interfering with the use of the catheter lumens or requiring exposure of the proximal end of the catheter lumen to the atmosphere.

There is a provided in a first embodiment of the present invention an infection management system according to claim 1. In this system, a catheter includes a side-arm tube which extends laterally from the side of the catheter, wherein the side-arm tube's lumen communicates with the catheter's lumen, and a one-way valve which prevents fluid flow from the catheter lumen into the side-arm tube lumen without preventing fluid flow through the catheter lumen. The side-arm tube is located in a region of the catheter which remains outside a patient's body when the catheter is in place within the patient, and between the patient's body and a proximal location at which the catheter may be clamped or pinched to shut off fluid flow out of or into the catheter. Once the catheter is within the patient, in order to provide antimicrobial agent-based, for example iodine-based, nosocomial infection management, an antimicrobial agent-bearing anti-infection device, for example an iodine-impregnated carrier such as an iodine-impregnated flexible rod or fiber, is inserted through the side-arm tube lumen and the one-way valve and passed into and through the catheter lumen. Once in place within the indwelling catheter, the anti-microbial agent, e.g. iodine, is released from the anti-infection device, e.g. the iodine-impregnated rod, in response to the local environmental conditions within the catheter. The anti-microbial agent, e.g. iodine, then may diffuse both within the catheter and through the semi-permeable catheter wall to the outside surface of the catheter to combat nosocomial infections.

A significant advantage of this system is that it permits the anti-infection device, e.g. iodine-bearing rod, to be inserted or removed from the catheter without interfering with fluid passage through the catheter lumen. For example, the anti-infection device can be inserted or removed without inhibiting the flow of blood being removed and replaced in the patient during hemodialysis. This embodiment of the present invention further avoids undesired exposure of the fluids within the catheter to the atmosphere, thus avoiding problems such as clot formation within the catheter lumen. This embodiment also avoids undesired bleeding out of fluids from the catheter's proximal end that can occur when a clamp along the catheter must be released in order to permit an anti-infection device such as an iodine-bearing device to be inserted into or removed from the catheter.

A further embodiment of the present invention employs a catheter that includes an extension tube connected to an outlet end of the catheter body. The extension tube may be removably joined to the rest of the catheter body to facilitate changing of connections to the indwelling portion of the catheter. In this embodiment, the side tube and one-way valve may be located in either the extension tube portion of the catheter or in the catheter body between the extension tube and the patient, as long as the side tube remains between the patient and the location at which the catheter may be pinched to shut off fluid flow to or from the catheter.

An infection management method is also disclosed for illustration, but not falling within the scope of the present invention. In this method, a catheter is provided, having a side arm tube whose lumen communicates with a lumen through the catheter. A one-way valve prevents fluid flow from the catheter lumen into the side arm tube lumen. An antimicrobial agent-bearing, e.g. iodine-bearing, carrier, which may be in the form of a flexible rod, is inserted through the side arm tube and the one-way valve until it is inserted a desired distance into and through the catheter. The anti-microbial agent may be released from the rod to enter the fluid within the catheter and to diffuse through the catheter wall.

Certain embodiments of the present invention provide an apparatus for management of catheter-related nosocomial infections with administration of an antimicrobial agent such as iodine in a manner that: (1) eliminates any need to open the end of the catheter, such as a hemodialysis catheter, to the atmosphere to either insert or remove an anti-infection device; (2) prevents back-bleeding through the catheter during anti-infection device insertion or removal; (3) allows the anti-infection device to remain in place during fluid flow, such as during hemodialysis; and/or (4) avoids any difficulty in anti-infection device insertion or removal as a result of the need to pass through regions of the catheter which have been clamped shut to stem fluid flow.

### Brief Description Of The Drawings

The foregoing and further objects, features and advantages of the invention will become apparent from the following detailed description with reference to the accompanying drawings, wherein like numerals are used to represent like elements and wherein:

Fig. 1 is a side view of a portion of a catheter showing a prior approach to delivery of an infection intervention device into a catheter lumen.

Fig. 2 is a side view of a catheter and side tube extension in accordance with an embodiment of the present invention.

Fig. 3 is a side view of an antimicrobial agent-bearing element to be inserted through a catheter in accordance with an embodiment of the present invention.

Fig. 4 is a side view of a catheter and side tube extension in accordance with another embodiment of the present invention.

### Detailed Description

Some possible embodiments of the invention, which is defined by claim 1, are hereafter described. A first embodiment of the present invention is illustrated in Fig. 2. In this embodiment catheter 8 includes a catheter body 9 whose distal end may be placed within a patient's body (not shown). Joined to the proximal end of catheter body 9 is an extension tube 10 with a lumen in communication with a lumen extending through the catheter body. Catheter body 9 may be inserted directly into the patient's body, or alternatively, may be removably coupled at its distal end to a catheter previously implanted in the patient. Extension tube 10 may be in turn joined to tubing 11 that is connected to, for example, medical equipment such as a hemodialysis machine (not shown). Any of a number of well-known methods of joining extension tube 10 and tubing 11 may be employed, such as the threaded barrel 12 shown in Fig. 2 which engages threads on the proximal end of extension tube 10.

At a side of extension tube 10 is a side arm tube 13. Side arm tube 13 may be joined to extension 10 in any conventional manner, such as by adhesive, integral molding with extension tube 10, welding, interference fit or threaded attachment, as long as a lumen within side arm tube 13 communicates through extension tube 10 with a lumen extending through catheter body 9. In addition to the side arm tube, this embodiment includes a one-way valve 14 which permits an iodine-based antimicrobial agent-bearing intervention device, in this embodiment, iodine-bearing flexible rod 15, to be inserted and removed through side arm tube 13 into and out of the catheter lumen, while preventing back flow of fluid from the catheter lumen through side arm tube 13. One-way valve 14 may be formed, for example, in a diaphragm shape from a self-sealing bio-compatible material which conforms to the exterior surface of iodine-bearing flexible rod 15 as it penetrates one-way valve 14, such as silicone, polyurethane or ethyl vinyl acetate. Other such one-way valve configurations suitable for this application will be readily apparent to those of skill in the art.

While certain examples make reference to iodine, it should be noted that the embodiments of the invention are not so limited. Indeed, any suitable antimicrobial agent can be used without parting from the scope of the principles described herein. Notwithstanding, there are a number of aspects of iodine-based techniques for which the embodiments are well suited.

Iodine-bearing flexible rod 15 may comprise a bio-compatible polymer material, such as a polycarbonate, urethane, ethyl, vinyl acetate, nylon, etc., adapted to carry the iodine to be released when the iodine-bearing rod is inserted through catheter 8. In addition iodine-bearing flexible rod 15 is affixed at its proximal end to a side arm tube end cap 16, as illustrated in Fig. 3.
Attachment to cap 16 permits an operator to avoid directly touching the proximal end of iodine-bearing flexible rod 15 while inserting or removing the rod through the catheter. In this embodiment, cap 16 contains internal threads which cooperate with external threads about the proximal end of side arm tube 13 (not shown) to seal side arm tube 13 when iodine-bearing flexible rod 15 is inserted into the catheter. Other cap engagement configurations may alternatively be used, as long as cap 16 effectively seals side arm tube 13, such as a stopper which frictionally engages the inner or outer surfaces of the side arm tube, an externally threaded cap, or bayonet features that cooperate with corresponding features on the end of side arm tube 13.

Referring again to Fig. 2, there is shown a clamp 17 positioned on extension tube 10 proximally from side arm tube 13. Clamp 17 may be used to throttle or completely halt fluid flow into or out of extension tube 10 as needed, for example, during its connection and disconnection from tubing associated with, for example, dialysis or infusion therapy equipment. Alternatively, clamp 17 may be a conventional valve, such as a ball valve or a gate valve. Because clamp 17 is located proximal to side arm tube 13, iodine-bearing flexible rod 15 may be inserted into or removed from the catheter without consideration of the status of the clamp, *i.e*., whether the clamp is open or closed. Thus, unlike the prior catheter shown in Fig. 1, the insertion or removal of iodine-bearing flexible rod 15 in the embodiment of the present invention catheter system shown in Fig. 2 is now independent of events associated with connection and disconnection of tubes to and from the proximal end of the catheter. Further, because clamp 17 no longer needs to be opened to permit the iodine-bearing intervention device to be inserted or removed from the catheter, unnecessary exposure of the catheter lumen to the atmosphere or back-bleeding of fluid from the catheter while clamp 17 is open is avoided.

It will be appreciated by persons of ordinary skill in the art that the wall of the catheter within the patient's body may be semi-permeable, allowing passage of iodine released from the iodine-bearing intervention device from the catheter lumen to an outer surface of the catheter. For example, the wall of the catheter lumen may be constructed of polyurethane, polycarbothane or ethyl vinyl acetate.

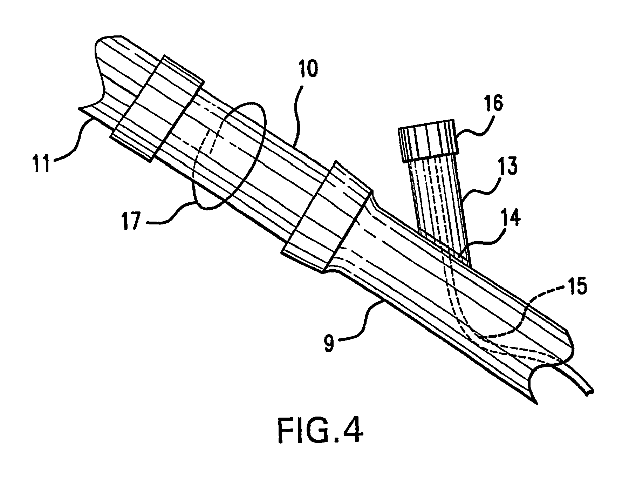

A further embodiment of the present invention is illustrated in Fig. 4. In this embodiment, side arm tube 13 is located in a portion of catheter body 9 between the point at which the catheter enters the patient's body (not shown) and a coupling joining catheter body 9 to extension tube 10. As in the previously discussed embodiment, iodine-bearing flexible rod 15, attached to cap 16 at its proximal end, is inserted and removed through side arm tube 13 and one-way valve 14 through the catheter lumen. In Fig. 4, clamp 17 is shown located on extension tube 10; however, it should be readily apparent that the clamp may be located on catheter body 9, for example, proximal to side arm tube 13 relative to the patient's body.

The following illustrative example shows a method of use of a catheter system to provide nosocomial infection management. In the first step of this method, there is provided a catheter with a side-arm tube extending laterally from a side of the catheter, such as the catheter and side arm tube in the embodiments illustrated in Fig. 2 and Fig. 4. The catheter also includes a one-way valve located within, or at the distal end of, the side arm tube, which prevents fluid flow from the catheter lumen into or out of the side-arm tube lumen. Following insertion of the distal end of the catheter body into the patient, or alternatively, coupling of the distal end of the catheter body to a previously implanted catheter, there is the step of inserting the antimicrobial agent -bearing intervention device, in one embodiment an iodine-bearing flexible polycarbonate rod, through the side-arm tube and the one-way valve into the catheter lumen. This method offers a greatly simplified approach to iodine-based anti-nosocomial infection management by eliminating the need to coordinate intervention device insertion and removal with other medical procedures employing the catheter.

While the present invention has been described with reference to what are presently considered to be preferred embodiments thereof, it is to be understood that the present invention is not limited to the disclosed embodiments or constructions. On the contrary, the present invention is intended to cover various modifications, that are also within the scope of the present invention.

## Claims

1. An infection management system, comprising:
a catheter (8) with a lumen extending therethrough;
a side-arm tube (13) extending laterally from a side of the catheter (8), wherein
the side-arm tube (13) is located in a region of the catheter (8) which remains outside a patient's body, and
a lumen through the side-arm tube communicates with the catheter lumen;
a one-way valve (14) which prevents fluid flow from the catheter lumen through the side-arm tube lumen without preventing fluid flow through the catheter lumen, **characterized in that** the infection management system further comprises:
an antimicrobial agent-bearing intervention device (15)
inserted through the side-arm tube lumen and the one-way valve (14) into the catheter lumen; and
a cap (16), wherein the antimicrobial agent-bearing intervention device (15) is an antimicrobial agent-bearing rod affixed to the cap (16), and the cap (16) seals a proximal end of the side-arm tube 13).

2. The infection management system of claim 1, **characterized in that**
the catheter (8) comprises a catheter body (9) and an extension (10) joined to a proximal end of the catheter body (9),
the extension (10) having a lumen extending longitudinally therethrough and in communication with the catheter lumen, and
the side-arm tube (13) extends laterally from the catheter extension (10).

3. The infection management system of claim 3, **characterized in that** the antimicrobial agent-bearing rod comprises a flexible polymer rod.

4. The infection management system of claim 1, wherein **characterized in that** the antimicrobial agent is iodine.

5. The infection management system of claim 1, **characterized in that** a region of the catheter (8) to be located within the patient's body comprises a material which permits passage of an antimicrobial agent released from the antimicrobial agent-bearing intervention device (15) from the catheter lumen to an outer surface of the catheter.

6. The infection management system of claim 1, **characterized in that** the cap (16) has a threaded portion that cooperates with a threaded portion on the side-arm tube (14).

7. The infection management system of claim 1, wherein the cap (16) is stopper sized to frictionally engage and seal the proximal end of the side-arm tube (14).

## Patentansprüche

1. Infektionsmanagementsystem, umfassend:
einen Katheter (8) mit einem Lumen, das sich durch diesen erstreckt,
ein Seitenarmrohr (13), das sich seitlich von einer Seite des Katheters (8) erstreckt, wobei
das Seitenarmrohr (13) in einem Bereich des Katheters (8) angeordnet ist, der außerhalb eines Patientenkörpers bleibt, und
ein Lumen durch das Seitenarmrohr mit dem Lumen des Katheters in Verbindung steht,
ein Einwegventil (14), das den Flüssigkeitsfluß vom Lumen des Katheters durch das Lumen des Seitenarmrohrs verhindert, ohne den Flüssigkeitsfluß durch den Lumen des Katheters zu verhindern,
**dadurch gekennzeichnet, daß** das Infektionsmanagementsystem ferner umfaßt:
eine ein antimikrobielles Mittel tragende Interventionsvorrichtung (15), die durch das Lumen des Seitenarmrohres und das Einwegventil (14) in das Lumen des Katheters eingeführt ist und
eine Kappe (16), wobei die das antimikrobielle Mittel-tragende Interventionsvorrichtung (15) ein ein antimikrobielles Mittel tragender Stab ist, der an der Kappe (16) befestigt ist, und die Kappe (16) ein proximales Ende des Seitenarmrohrs (13) abdichtet.

2. Infektionsmanagementsystem nach Anspruch 1, **dadurch gekennzeichnet, daß**
der Katheter (8) einen Katheterkörper (9) und eine mit einem proximalen Ende des Katheterkörpers (9) verbundene Verlängerung (10) umfaßt,
die Verlängerung (10) ein Lumen aufweist, das sich durch diese in Längsrichtung erstreckt und in Verbindung mit dem Lumen des Katheters steht, und
das Seitenarmrohr (13) sich seitlich von der Katheterverlängerung (10) erstreckt.

3. Infektionsmanagementsystem nach Anspruch 2, **dadurch gekennzeichnet, daß** der das antimikrobielle Mittel tragende Stab einen biegsamen Polymerstab umfaßt.

4. Infektionsmanagementsystem nach Anspruch 1, **dadurch gekennzeichnet, daß** das antimikrobielle Mittel Jod ist.

5. Infektionsmanagementsystem nach Anspruch 1, **dadurch gekennzeichnet, daß** ein Bereich des Katheters (8), der innerhalb eines Patientenkörpers angeordnet wird, ein Material umfaßt, das den Übergang eines von der das antimikrobielle Mittel tragenden Interventionsvorrichtung (15) freigesetzten antimikrobiellen Mittel von dem Lumen des Katheters zu einer äußeren Oberfläche des Katheters erlaubt.

6. Infektionsmanagementsystem nach Anspruch 1, **dadurch gekennzeichnet, daß** die Kappe (16) einen Gewindeabschnitt aufweist, der mit einem Gewindeabschnitt an dem Seitenarmrohr (14) zusammenwirkt.

7. Infektionsmanagementsystem nach Anspruch 1, wobei die Kappe (16) stöpselgroß ist, um das proximale Endes des Seitenarmrohres (14) reibschlüssig zu belegen und abzudichten.

## Revendications

1. Système de gestion d'infection, comprenant :
un cathéter (8) avec une lumière s'étendant à travers ce dernier ;
un tube de dérivation (13) s'étendant latéralement à partir d'un côté du cathéter (8), dans lequel :
le tube de dérivation (13) est positionné dans une région du cathéter (8) qui reste à l'extérieur du corps d'un patient, et
une lumière à travers le tube de dérivation communique avec la lumière de cathéter ;
une soupape à une voie (14) qui empêche l'écoulement de fluide de la lumière de cathéter par la lumière du tube de dérivation sans empêcher l'écoulement de fluide à travers la lumière de cathéter, **caractérisé en ce que** le système de gestion d'infection comprend en outre :
un dispositif d'intervention de support d'agent antimicrobien (15) inséré à travers la lumière du tube de dérivation et la soupape à une voie (14) dans la lumière de cathéter ; et
un capuchon (16), dans lequel le dispositif d'intervention de support d'agent antimicrobien (15) est une tige du support d'agent antimicrobien fixée sur le capuchon (16) et le capuchon (16) réalise l'étanchéité d'une extrémité proximale du tube de dérivation (13).

2. Système de gestion d'infection selon la revendication 1, **caractérisé en ce que** :
le cathéter (8) comprend un corps de cathéter (9) et une extension (10) assemblée à une extrémité proximale du corps de cathéter (9),
l'extension (10) ayant une lumière s'étendant de manière longitudinale à travers cette dernière et en communication avec la lumière de cathéter, et
le tube de dérivation (13) s'étend de manière latérale à partir de l'extension de cathéter (10).

3. Système de gestion d'infection selon la revendication 3, **caractérisé en ce que** la tige de support d'agent antimicrobien comprend une tige en polymère souple.

4. Système de gestion d'infection selon la revendication 1, **caractérisé en ce que** l'agent antimicrobien est de l'iode.

5. Système de gestion d'infection selon la revendication 1, **caractérisé en ce qu'**une région du cathéter (8) destinée à être positionnée dans le corps du patient comprend un matériau qui permet le passage d'un agent antimicrobien libéré par le dispositif d'intervention de support d'agent antimicrobien (15) de la lumière de cathéter jusqu'à une surface externe du cathéter.

6. Système de gestion d'infection selon la revendication 1, **caractérisé en ce que** le capuchon (16) a une partie filetée qui coopère avec une partie filetée du tube de dérivation (14).

7. Système de gestion d'infection selon la revendication 1, dans lequel le capuchon (16) est une butée dimensionnée pour mettre en prise par frottement et réaliser l'étanchéité de l'extrémité proximale du tube de dérivation (14).
